# FASCICULE DE BREVET EUROPEEN

(11) **EP 3 189 827 B1**
(45) Date de publication et mention de la délivrance du brevet: **21.11.2018**
(21) Numéro de dépôt: 17150557.1
(22) Date de dépôt: 06.01.2017
(51) Int. Cl.: A61K 8/26, A61Q 13/00, A61K 8/73, A61K 47/00, A61K 9/107, A61K 8/06, A61P 17/02

(54) **FORMULATION AQUEUSE COMPRENANT UNE COMPOSITION LIPOPHILE COMPRENANT DE LA CELLULOSE ET DE L'ARGILE**
WÄSSRIGE FORMULIERUNG, DIE EINE LIPOPHILE ZUSAMMENSETZUNG UMFASST, DIE CELLULOSE UND TON UMFASST
AQUEOUS FORMULATION COMPRISING A LIPOPHILIC COMPOSITION COMPRISING CELLULOSE AND CLAY

(30) Priorité: 08.01.2016 FR 1650158
(43) Date de publication de la demande: 12.07.2017
(73) Titulaire: Evergreen Land Limited, Central Hong Kong (HK)
(72) Inventeur: VIDAL, Nicolas, 13012 Marseille (FR); LESGARDS, Jean-François, 13008 Marseille (FR)
(74) Mandataire: Sayettat, Julien Christian

(56) Documents cités:
- WO-A1-2005/051339

## Description

L'invention concerne une formulation aqueuse comprenant entre 0,1 et 50% en poids d'une composition lipophile, par exemple une huile essentielle ou un mélange d'huiles essentielles.

Elle s'applique notamment à une formulation aqueuse à usage cosmétique et/ou pharmaceutique, la composition lipophile, par exemple une huile essentielle ou un mélange d'huiles essentielles, présentant alors des propriétés correspondant à l'effet cosmétique et/ou pharmaceutique recherché, par exemple une fonction répulsive pour des animaux nuisibles, une fonction thérapeutique et/ou prophylactique, par exemple une fonction apaisante destinée à soulager les piqûres ou morsures d'animaux nuisibles, une fonction anti brûlures, une fonction cicatrisante, une fonction relaxante ou une fonction anti-inflammatoire, ou encore une fonction de bien-être comme une fonction parfumante, une fonction déodorante, une fonction biocide, une fonction bactéricide ou une fonction fongicide.

De telles formulations sont généralement destinées à être utilisées soit par application directe sur la peau d'un sujet, soit de manière indirecte dans l'air ou sur d'autres surfaces, par exemple au moyen d'un pulvérisateur, d'un applicateur à bille ou d'autres modes d'application.

L'eau et les compositions lipophiles étant des liquides non miscibles, il est connu d'utiliser des agents émulsifiants, notamment synthétiques, pour assurer la stabilité de leur mélange, d'autant plus lorsque la concentration en composition lipophile est importante.

Toutefois, la nature synthétique de ces agents émulsifiants pose problème, notamment dans le cadre d'une utilisation cosmétique et/ou pharmaceutique de la formulation. En effet, leur présence dans la formulation peut altérer les propriétés de la composition lipophile, notamment les propriétés parfumantes, mais également provoquer des réactions cutanées indésirables, notamment des irritations ou des allergies, chez certains sujets.

Pour pallier ces inconvénients, on connaît du document JP-S55/162710 une formulation aqueuse à usage cosmétique comprenant de l'eau et une forte concentration (jusqu'à 85% en poids) en huile(s), ainsi qu'un mélange homogénéisant comprenant notamment de la cellulose microcristalline, éventuellement complétée avec un polymère soluble dans l'eau, par exemple à base de carboxymethylcellulose, pour limiter le plus possible l'utilisation d'agents émulsifiants synthétiques.

Toutefois, cette formulation ne donne pas entière satisfaction, en ce qu'elle ne permet pas de stabiliser complètement le mélange eau-huile(s). De ce fait, la formulation peut, au bout d'un certain temps, présenter un déphasage entre l'eau et la(les) huile(s), ce qui peut entraîner des problèmes de répartition de l'élément actif formé par la(les)dite(s) huile(s) au sein du contenant de ladite formulation et/ou sur la partie du corps du sujet sur laquelle ladite formulation est appliquée, et ainsi nuire à l'efficacité de ladite formulation.

Pour améliorer la stabilité, notamment dans le temps, d'une formulation aqueuse comprenant une forte concentration en huile(s), le document FR-2 974 312 propose d'ajouter à une telle formulation des nanocristaux de cellulose, mais cette solution s'avère relativement complexe à mettre en oeuvre.

Par ailleurs, on connaît du document EP-1 699 429 une formulation aqueuse à usage cosmétique comprenant de l'eau et une forte concentration (au moins 30% en poids) en composition lipophile à base d'huile(s) parfumée(s), notamment une (des) huile(s) essentielle(s), ainsi qu'une petite quantité (entre 0,1 et 4% en poids) d'un dérivé de cellulose, notamment le carboxymethylcellulose et/ou d'une argile, notamment de la bentonite.

Toutefois, dans ce document, l'ajout de bentonite et/ou du dérivé de cellulose vise principalement à épaissir la formulation, alors que la stabilisation de la formulation aqueuse est obtenue par l'ajout d'un agent émulsifiant. Par ailleurs, la quantité d'agent épaississant rend la formulation difficile à appliquer, notamment au moyen d'un pulvérisateur.

L'invention vise à perfectionner l'art antérieur en proposant notamment une formulation aqueuse comprenant de l'eau et une forte concentration en composition lipophile, dans laquelle le mélange eau-composition lipophile présente une homogénéité et une stabilité dans le temps améliorées tout en étant facile à appliquer, notamment à l'aide d'un pulvérisateur ou d'un applicateur à bille, sur la peau d'un sujet, ladite formulation étant en outre facile à fabriquer.

A cet effet, l'invention propose une formulation aqueuse comprenant entre 0,1 et 50% en poids d'une composition lipophile, ladite formulation comprenant en outre :
- entre 0,1 et 10% en poids d'un composant homogénéisant comprenant au moins de la cellulose microcristalline ;
- entre 0,01 et 2% en poids d'une argile ;
- le complément en eau.

D'autres particularités et avantages de l'invention apparaîtront dans la description qui suit de différents modes de réalisation particuliers.

L'invention concerne une formulation comprenant entre 0,1% et 50% en poids d'une composition lipophile, ladite composition lipophile comprenant notamment au moins une huile, et plus particulièrement une huile essentielle ou un mélange d'huiles essentielles. En particulier, l'invention concerne une formulation comprenant une forte concentration, notamment plus de 20% en poids, d'une composition lipophile.

L'invention s'applique notamment à une formulation aqueuse à usage cosmétique et/ou pharmaceutique, la composition lipophile présentant alors des propriétés correspondant à l'effet cosmétique et/ou pharmaceutique recherché.

En particulier, la composition lipophile peut présenter une fonction répulsive pour des animaux nuisibles. A cet effet, la composition lipophile peut par exemple comprendre du p-menthane-3,8-diol (PMD), qui est un ingrédient naturel particulièrement efficace pour repousser les insectes hématophages piqueurs ou mordeurs tels que les moustiques ou les tiques. Selon une réalisation, au moins 10%, et notamment plus de 40%, en poids de PMD sont présents dans la composition lipophile.

A titre d'exemple, la composition lipophile peut comprendre de l'huile essentielle dérivée d'eucalyptus citronné ou de citronnelle, telle que par exemple les huiles essentielles commercialisées sous les marques Citrepel® et Citriodiol®, de telles huiles constituant des sources naturelles connues de p-menthane-3,8-diol.

La composition lipophile peut également présenter d'autres fonctions, notamment des fonctions thérapeutiques et/ou prophylactiques comme des fonctions apaisantes destinées à soulager les piqûres ou morsures d'animaux nuisibles, des fonctions anti-brûlures, cicatrisantes, relaxantes ou anti-inflammatoire, ou encore des fonctions de bien-être comme des fonctions parfumantes, déodorantes, biocides, bactéricides ou fongicides.

La formulation aqueuse comprend en outre entre 0,1 et 10% en poids, et notamment entre 0,1 et 2% en poids, d'un composant homogénéisant comprenant au moins de la cellulose microcristalline, afin d'améliorer la répartition de la composition lipophile dans l'eau.

En particulier, la cellulose microcristalline est un ingrédient d'origine naturelle, notamment issue du bois, qui est organisée en faisceaux à l'échelle macroscopique. Par ailleurs, elle est insoluble dans l'eau, et forme un gel en générant des liaisons solide-solide entre l'eau et la composition lipophile lors de son ajout à la formulation aqueuse.

Selon un mode de réalisation, le composant homogénéisant comprend en outre de la carboxyméthylcellulose, de la gomme xanthane, ou un mélange de ces composés, la gomme xanthane présentant notamment des propriétés épaississantes et gélifiantes. En particulier, la cellulose microcristalline peut être co-processée avec la carboxyméthylcellulose et/ou avec la gomme xanthane lors de la fabrication du composant homogénéisant.

De façon particulièrement avantageuse, le composant homogénéisant comprend un mélange de cellulose microcristalline avec de la carboxyméthylcellulose et/ou de la gomme xanthane, ledit mélange comprenant notamment entre 75 et 95% en poids de cellulose microcristalline et entre 5 et 25% en poids de carboxyméthylcellulose et/ou de gomme xanthane.

En outre, la formulation aqueuse comprend entre 0,01 et 2% en poids, notamment moins de 1% en poids, et plus particulièrement moins de 0,5% en poids, d'une argile, afin de stabiliser dans le temps le mélange eau-composition lipophile, et ainsi d'éviter le déphasage et les problèmes d'homogénéité et de répartition de la composition lipophile qui peuvent en découler, notamment durant l'application de ladite formulation sur la peau d'un sujet. Par ailleurs, la faible quantité d'argile permet de limiter l'épaississement de la formulation, et ainsi faciliter son application, notamment au moyen d'un pulvérisateur standard ou d'un applicateur à bille.

En particulier, l'argile peut comprendre une smectite, notamment en comprenant de la bentonite, ou d'autres familles d'argiles telles que la kaolinite, l'illite, la chlorite, la vermiculite, la sépiolite ou l'attapulgite. De façon avantageuse, l'argile peut comprendre de la bentonite avec une pureté proche de 100% en masse, et contenant moins de 10% en poids de silice cristalline totale.

La formulation aqueuse peut comprendre en outre d'autres ingrédients, notamment pour lui conférer des propriétés supplémentaires.

Ainsi, la formulation aqueuse peut comprendre entre 0,1 et 50%, notamment entre 5 et 20%, en poids d'une composition parfumante. En particulier, la composition parfumante peut comprendre une huile essentielle ou un mélange d'huiles essentielles, ou d'autres ingrédients notamment extraits d'huiles essentielles, comme des esters ou des terpènes.

Par exemple, la composition parfumante peut être à base d'Iceberg 57656® ou RS57656®.

Par ailleurs, la formulation peut également comprendre :
- moins de 20% en poids d'un agent conservateur, par exemple une quantité comprise entre 0,4 de 5% en poids d'alcool de phénéthyle d'origine naturelle ;
- moins de 10% en poids d'un agent antioxydant, par exemple une quantité inférieure à 1% en poids de vitamine E ;
- moins de 2% en poids d'un agent rectificateur de pH, notamment à base d'acide citrique, pour ajuster le pH de ladite formulation sur une plage comprise entre 3 et 7, préférablement proche du pH de la peau ;
- entre 3 et 50%, notamment moins de 10%, en poids d'un ou de plusieurs solvants, le(s)dit(s) solvant(s) étant non assimilable(s) à des composants lipophiles ou à des agents tensioactifs, et notamment choisi(s) parmi les alcools, les acides et/ou les sels d'acide. En particulier, le(s) solvant(s) peut (peuvent) être choisi(s) parmi l'éthanol, l'isopropanol, le méthylenediol, l'éthylène glycol, le propanediol, le glycérol, le butanediol, le pentanol, l'isopentyldiol, l'hexanol, l'hexanediol et le benzanediol.

Enfin, quelle que soit la composition de la formulation aqueuse, notamment en ce qui concerne les quantités de composition lipophile, de mélange homogénéisant, d'argile, et des autres ingrédients possibles, ladite formulation comprend le complément à 100% en eau, notamment de l'eau déminéralisée.

En particulier, la formulation aqueuse peut être fabriquée suivant un procédé simple, qui prévoit les étapes suivantes :
- mélange de la composition lipophile, du composant homogénéisant, de l'argile et des éventuels agents parfumants, conservateurs, antioxydant et/ou rectificateur de pH ;
- agitation mécanique dudit mélange, notamment au moyen d'un rotor-stator fonctionnant à une vitesse de 3000 tours/min, pendant une durée d'environ 10 minutes ;
- mise au repos de la formulation obtenue, pendant une durée comprise entre 10 et 30 minutes.

Par ailleurs, si la formulation aqueuse à obtenir comprend également un solvant, le procédé de fabrication peut prévoir, après la mise au repos susmentionnée, les étapes supplémentaires suivantes :
- ajout dudit solvant à ladite formulation ; et
- agitation mécanique de ladite formulation, notamment au moyen d'un rotor-stator fonctionnant à une vitesse de 3000 tours/min, pendant une durée d'environ 5 minutes.

### Exemples

Au cours d'une procédure de tests menée en laboratoire, neuf échantillons de formulations aqueuses ont été préparés, notamment suivant un procédé tel que décrit ci-dessus, et lesdits échantillons comprenaient tous de l'eau déminéralisée, ainsi que :
- 26% en poids d'une huile essentielle d'eucalyptus citronné de type Citrepel® ;
- 13% en poids d'une composition parfumante de type Iceberg 57656® ;
- 3,5% en poids de glycérol ;
- 0,25% en poids de vitamine E, commercialisée sous la marque Bioxan® ;
- jusqu'à 0,02% en poids d'acide citrique.

Par ailleurs, chacun de ces échantillons comprenait :
- pour les échantillons 1, correspondant à des échantillons test de la formulation proposée par l'invention :
   ∘ échantillon 1a : 1% en poids de cellulose microcristalline et 0,2% en poids de bentonite ;
   ∘ échantillon 1b :1% en poids d'un mélange homogénéisant à base de cellulose microcristalline co-processée avec de la carboxyméthylcellulose et 0,2% en poids de bentonite, ledit mélange homogénéisant comprenant environ 85% en poids de cellulose microcristalline et environ 15% en poids de carboxyméthylcellulose ;
- pour l'échantillon 2 : 0,2% en poids de bentonite ;
- pour l'échantillon 3 : 1% en poids d'un mélange homogénéisant à base de cellulose microcristalline et de carboxyméthylcellulose ;
- pour l'échantillon 4 : 1% en poids de gomme xanthane et 0,2% en poids de bentonite ;
- pour l'échantillon 5 : 1% en poids de carboxyméthylcellulose ;
- pour l'échantillon 6 : 1% en poids de carboxymethylcellulose et 0,2% en poids de bentonite ;
- pour l'échantillon 7 : 1% en poids d'un mélange homogénéisant à base de cellulose microcristalline et de gomme xanthane ;
- pour l'échantillon 8 : 11% en poids d'un mélange homogénéisant à base de cellulose microcristalline et de carboxymethylcellulose et 2,5% en poids de bentonite, ledit mélange homogénéisant comprenant environ 85% en poids de cellulose microcristalline et environ 15% en poids de carboxyméthylcellulose.

Ensuite, ces neuf échantillons ont tous émis soumis à des tests d'efficacité pour évaluer respectivement leur stabilité dans le temps, notamment par un test de déphasage à 54°C pendant 24H, et leur distribution par pulvérisation.

A la suite de ces tests, les résultats obtenus étaient les suivants :
- s'agissant du test de déphasage, seul les échantillons test 1a, 1b présentait des résultats satisfaisants ;
- s'agissant du test de distribution par pulvérisation, seuls les échantillons 1a, 1b, 2 et 3 présentaient des résultats satisfaisants.

Ainsi, ces tests ont permis de démontrer, du fait des bons résultats obtenus par les échantillons test 1a, 1b, l'efficacité de l'ajout simultané de bentonite et d'un mélange homogénéisant à base de cellulose microcristalline, et ce dans des quantités appropriées, pour garantir à la fois la stabilité dans le temps et une bonne distribution par pulvérisation d'une formulation aqueuse comprenant une forte concentration en composition lipophile.

En effet, les bons résultats des échantillons test 1a, 1b, tant en termes de stabilité dans le temps que de distribution par pulvérisation, ont montré l'efficacité de la présence combinée de cellulose microcristalline et de bentonite dans une formulation aqueuse riche en composition lipophile, notamment en comparaison avec les résultats obtenus par l'échantillon 5, qui contenait 1% de carboxyméthycellulose, et était notamment dépourvu de bentonite et de cellulose microcristalline.

En outre, les résultats obtenus par l'échantillon 2, qui contenait de la bentonite dans des proportions correspondant à celle de l'invention (0,2%), mais était dépourvu de cellulose microcristalline, ont montré que, si la présence de bentonite seule dans de telles proportions dans une formulation aqueuse riche en huile(s) essentielle(s) permettait d'obtenir des résultats satisfaisants en termes de distribution par pulvérisation, elle n'était cependant pas suffisante pour garantir une bonne stabilité dans le temps, dans la mesure où cette formulation présentait un déphasage eau-composition lipophile relativement important après le test de déphasage.

De même, les résultats obtenus par l'échantillon 3, qui contenait un mélange de cellulose microcristalline et de carboxyméthylcellulose dans des proportions correspondant à celle de l'invention (1%), mais était dépourvu de bentonite, ont montré que, si la présence de ce mélange seul dans de telles proportions dans une formulation aqueuse riche en composition lipophile permettait également, à l'instar de l'échantillon 2 contenant uniquement de la bentonite, d'obtenir de bons résultats en termes de distribution par pulvérisation, elle ne suffisait pas non plus pour garantir une bonne stabilité dans le temps.

Par ailleurs, les résultats obtenus par l'échantillon test 1b, qui contenait 1% d'un mélange cellulose microcristalline / carboxyméthylcellulose et 0,2% de bentonite, comparés à ceux obtenus par l'échantillon 6, qui contenait également 0,2% de bentonite, mais 1% d'un mélange homogénéisant uniquement à base de carboxyméthylcellulose, ont montré l'efficacité accrue de l'ajout de cellulose microcristalline dans des proportions judicieusement choisies, et ce tant en termes de distribution par pulvérisation que de stabilité dans le temps.

De plus, les résultats obtenus par l'échantillon test 1a, qui contenait 1% d'un mélange homogénéisant uniquement à base de cellulose microcristalline et 0,2% de bentonite, ont permis de montrer, par comparaison avec les résultats obtenus avec l'échantillon 6, l'efficacité de la simple combinaison cellulose microcristalline / bentonite, et ce dans des proportions judicieusement choisies, tant en termes de distribution par pulvérisation que de stabilité dans le temps.

En outre, les résultats obtenus par l'échantillon 4, qui contenaient de la bentonite combinée avec un ingrédient autre que la cellulose microcristalline (gomme xanthane), ainsi que les résultats obtenus par l'échantillon 7, qui contenait de la cellulose microcristalline combinée avec un ingrédient autre que la bentonite (gomme xanthane) ont montré l'efficacité accrue de la présence combinée de bentonite et de cellulose microcristalline par rapport à la présence combinée de l'un ou l'autre de ces ingrédients avec d'autres types d'ingrédients, tant en termes de stabilité dans le temps que de distribution par pulvérisation.

Enfin, les résultats obtenus par l'échantillon 8, qui contenait, tout comme les échantillons tests 1a, 1b, à la fois de la bentonite et un mélange homogénéisant à base de cellulose microcristalline, mais dans des proportions différentes, et notamment supérieures à celles dudit échantillon test (11% pour le mélange à base de cellulose microcristalline et 2,5% pour la bentonite), ont montré que la seule présence combinée de ces deux ingrédients dans une formulation aqueuse riche en composition lipophile n'était pas suffisante pour garantir une bonne stabilité dans le temps, ni une bonne distribution par pulvérisation, et que l'ajout de ces deux ingrédients devait nécessairement se faire avec des proportions judicieusement choisies pour garantir de bons résultats.

## Revendications

1. Formulation aqueuse comprenant entre 0,1 et 50% en poids d'une composition lipophile, ladite formulation étant **caractérisée en ce qu'**elle comprend en outre :
- entre 0,1 et 10% en poids d'un composant homogénéisant comprenant au moins de la cellulose microcristalline ;
- entre 0,01 et 2% en poids d'une argile ;
- le complément en eau.

2. Formulation aqueuse selon la revendication 1, **caractérisée en ce que** le composant homogénéisant comprend en outre de la carboxyméthylcellulose, de la gomme xanthane ou un mélange de ces composés.

3. Formulation aqueuse selon l'une des revendications 1 ou 2, **caractérisée en ce que** l'argile comprend une smectite, notamment de la bentonite.

4. Formulation aqueuse selon l'une quelconque des revendications 1 à 3, **caractérisée en ce que** l'argile comprend de la kaolinite, de l'illite, de la chlorite, de la vermiculite, de la sépiolite ou de l'attapulgite.

5. Formulation aqueuse selon l'une quelconque des revendications 1 à 4, **caractérisée en ce qu'**elle comprend plus de 20% en poids d'une composition lipophile.

6. Formulation aqueuse selon l'une quelconque des revendications 1 à 5, **caractérisée en ce que** la composition lipophile comprend au moins une huile, notamment une huile essentielle ou un mélange d'huiles essentielles.

7. Formulation aqueuse selon l'une quelconque des revendications 1 à 6, **caractérisée en ce que** la composition lipophile présente une fonction répulsive pour des animaux nuisibles.

8. Formulation aqueuse selon la revendication 6, **caractérisée en ce que** la composition lipophile comprend du p-menthane-3,8-Diol (PMD).

9. Formulation aqueuse selon l'une quelconque des revendications 1 à 8, **caractérisée en ce que** la composition lipophile présente au moins une fonction choisie parmi les fonctions thérapeutiques et/ou prophylactiques, notamment les fonctions apaisantes destinées à soulager les piqûres ou morsures d'animaux nuisibles, les fonctions anti-brûlures, cicatrisantes, relaxantes et anti-inflammatoires, et les fonctions de bien-être, notamment les fonctions parfumantes, déodorantes, biocides, bactéricides et fongicides.

10. Formulation aqueuse selon l'une quelconque des revendications 1 à 9, **caractérisée en ce qu'**elle comprend entre 0,1 et 2% en poids de composant homogénéisant.

11. Formulation aqueuse selon l'une quelconque des revendications 1 à 10, **caractérisée en ce que** le composant homogénéisant comprend un mélange de cellulose microcristalline et de carboxyméthylcellulose.

12. Formulation aqueuse selon l'une quelconque des revendications 1 à 11, **caractérisée en ce qu'**elle comprend moins de 1%, notamment moins de 0,5%, en poids d'une argile.

13. Formulation aqueuse selon l'une quelconque des revendications 1 à 12, **caractérisée en ce qu'**elle comprend entre 0,1 et 50%, notamment entre 5 et 20%, en poids d'une composition parfumante.

14. Formulation aqueuse selon la revendication 13, **caractérisée en ce que** la composition parfumante comprend une huile essentielle ou un mélange d'huiles essentielles.

15. Formulation aqueuse selon l'une quelconque des revendications 1 à 14, **caractérisée en ce qu'**elle comprend en outre moins de 20% en poids d'un agent conservateur.

16. Formulation aqueuse selon la revendication 15, **caractérisée en ce que** l'agent conservateur comprend de l'alcool de phénéthyle d'origine naturelle, notamment en une quantité comprise entre 0,4 de 5% en poids de ladite formulation.

17. Formulation aqueuse selon l'une quelconque des revendications 1 à 16, **caractérisée en ce qu'**elle comprend en outre moins de 10% en poids d'un agent antioxydant.

18. Formulation aqueuse selon la revendication 17, **caractérisée en ce que** l'agent antioxydant comprend de la vitamine E, notamment en une quantité inférieure à 1% en poids de ladite formulation.

19. Formulation aqueuse selon l'une quelconque des revendications 1 à 18, **caractérisée en ce qu'**elle comprend en outre moins de 2% en poids d'un agent rectificateur de pH, notamment à base d'acide citrique.

20. Formulation aqueuse selon l'une quelconque des revendications 1 à 19, **caractérisée en ce qu'**elle comprend en outre entre 3 et 50%, notamment moins de 10%, en poids d'un ou de plusieurs solvant(s), notamment choisi(s) parmi les alcools, les acides et/ou les sels d'acides.

21. Formulation aqueuse selon la revendication 20, **caractérisé en ce que** le(s) solvant(s) est (sont) choisi(s) parmi l'éthanol, l'isopropanol, le méthylenediol, l'éthylène glycol, le propanediol, le glycérol, le butanediol, le pentanol, l'isopentyldiol, l'hexanol, l'hexanediol et le benzanediol.

## Patentansprüche

1. Wässrige Formulierung, umfassend zwischen 0,1 und 50 Gewichts-% eine lipophile Zusammensetzung, wobei die Formulierung **dadurch gekennzeichnet ist, dass** sie des Weiteren Folgendes umfasst:
- zwischen 0,1 und 10 Gewichts-% eines homogenisierenden Bestandteils, umfassend mindestens mikrokristalline Cellulose;
- zwischen 0,01 und 2 Gewichts-% einer Tonerde;
- den Rest Wasser.

2. Wässrige Formulierung nach Anspruch 1, **dadurch gekennzeichnet, dass** der homogenisierende Bestandteil des Weiteren Carboxymethylcellulose, Xanthangummi oder eine Mischung dieser Verbindungen umfasst.

3. Wässrige Formulierung nach einem der Ansprüche 1 oder 2, **dadurch gekennzeichnet, dass** die Tonerde einen Smektit umfasst, insbesondere Bentonit.

4. Wässrige Formulierung nach einem der Ansprüche 1 bis 3, **dadurch gekennzeichnet, dass** die Tonerde Kaolinit, Illit, Chlorit, Vermikulit, Sepiolith oder Attapulgit umfasst.

5. Wässrige Formulierung nach einem der Ansprüche 1 bis 4, **dadurch gekennzeichnet, dass** sie mehr als 20 Gewichts-% einer lipophilen Zusammensetzung umfasst.

6. Wässrige Formulierung nach einem der Ansprüche 1 bis 5, **dadurch gekennzeichnet, dass** die lipophile Zusammensetzung mindestens ein Öl umfasst, insbesondere ein ätherisches Öl oder eine Mischung von ätherischen Ölen.

7. Wässrige Formulierung nach einem der Ansprüche 1 bis 6, **dadurch gekennzeichnet, dass** die lipophile Zusammensetzung eine abstoßende Funktion für tierische Schädlinge aufweist.

8. Wässrige Formulierung nach Anspruch 6, **dadurch gekennzeichnet, dass** die lipophile Zusammensetzung p-Menthan-3,8-diol (PMD) umfasst.

9. Wässrige Formulierung nach einem der Ansprüche 1 bis 8, **dadurch gekennzeichnet, dass** die lipophile Zusammensetzung mindestens eine Funktion aufweist, ausgewählt aus therapeutischen und/oder vorbeugenden Funktionen, insbesondere beruhigenden Funktionen bestimmt zum Lindern von Stichen oder Bissen tierischer Schädlinge, Verbrennungsschutz-, Wundheilungs-, Entspannungs- und Entzündungshemmfunktionen, und Funktionen des Wohlbefindens, insbesondere Parfümierungs-, Deodorierungs-, Biozid-, Bakterizid- und Fungizidfunktionen.

10. Wässrige Formulierung nach einem der Ansprüche 1 bis 9, **dadurch gekennzeichnet, dass** sie zwischen 0,1 und 2 Gewichts-% homogenisierenden Bestandteil umfasst.

11. Wässrige Formulierung nach einem der Ansprüche 1 bis 10, **dadurch gekennzeichnet, dass** der homogenisierende Bestandteil eine Mischung aus mikrokristalliner Cellulose und Carboxymethylcellulose umfasst.

12. Wässrige Formulierung nach einem der Ansprüche 1 bis 11, **dadurch gekennzeichnet, dass** sie weniger als 1 Gewichts-%, insbesondere weniger als 0,5 Gewichts-%, einer Tonerde umfasst.

13. Wässrige Formulierung nach einem der Ansprüche 1 bis 12, **dadurch gekennzeichnet, dass** sie zwischen 0,1 und 50 Gewichts-%, insbesondere zwischen 5 und 20 Gewichts-%, eine Parfümzusammensetzung umfasst.

14. Wässrige Formulierung nach Anspruch 13, **dadurch gekennzeichnet, dass** die Parfümzusammensetzung ein ätherisches Öl oder eine Mischung von ätherischen Ölen umfasst.

15. Wässrige Formulierung nach einem der Ansprüche 1 bis 14, **dadurch gekennzeichnet, dass** sie des Weiteren weniger als 20 Gewichts-% eines Konservierungsmittels umfasst.

16. Wässrige Formulierung nach Anspruch 15, **dadurch gekennzeichnet, dass** das Konservierungsmittel Phenylethanol natürlichen Ursprungs umfasst, insbesondere in einer Menge im Bereich zwischen 0,4 und 5 Gewichts-% der Formulierung.

17. Wässrige Formulierung nach einem der Ansprüche 1 bis 16, **dadurch gekennzeichnet, dass** sie des Weiteren weniger als 10 Gewichts-% eines Antioxidationsmittels umfasst.

18. Wässrige Formulierung nach Anspruch 17, **dadurch gekennzeichnet, dass** das Antioxidationsmittel Vitamin E umfasst, insbesondere in einer Menge unterhalb von 1 Gewichts-% der Formulierung.

19. Wässrige Formulierung nach einem der Ansprüche 1 bis 18, **dadurch gekennzeichnet, dass** sie des Weiteren weniger als 2 Gewichts-% eines pH-Rektifizierungsmittels umfasst, insbesondere auf Basis von Zitronensäure.

20. Wässrige Formulierung nach einem der Ansprüche 1 bis 19, **dadurch gekennzeichnet, dass** sie des Weiteren zwischen 3 und 50 Gewichts-%, insbesondere weniger als 10 Gewichts-%, eines oder mehrere Lösemittel umfasst, insbesondere ausgewählt aus Alkoholen, Säuren und/oder Salzen von Säuren.

21. Wässrige Formulierung nach Anspruch 20, **dadurch gekennzeichnet, dass** das/die Lösemittel ausgewählt ist/sind aus Ethanol, Isopropanol, Methylendiol, Ethylenglycol, Propandiol, Glycerol, Butandiol, Pentanol, Isopentyldiol, Hexanol, Hexandiol und Dihydroxybenzol.

## Claims

1. Aqueous formulation comprising between 0.1% and 50% by weight of lipophilic composition, said formulation being **characterised in that** it further comprises:
- between 0.1% and 10% by weight of homogenising component comprising at least microcrystalline cellulose;
- between 0.01% and 2% by weight of clay;
- the remainder in water.

2. Aqueous formulation according to claim 1, **characterised in that** the homogenising component further comprises carboxymethylcellulose, xanthan gum or a mixture of these compounds.

3. Aqueous formulation according to one of claims 1 or 2, **characterised in that** the clay comprises a smectite, in particular bentonite.

4. Aqueous formulation according to any of claims 1 to 3, **characterised in that** the clay comprises kaolinite, illite, chlorite, vermiculite, sepiolite or attapulgite.

5. Aqueous formulation according to any of claims 1 to 4, **characterised in that** it comprises more than 20% by weight of lipophilic composition.

6. Aqueous formulation according to any of claims 1 to 5, **characterised in that** the lipophilic composition comprises at least one oil, in particular an essential oil or a mixture of essential oils.

7. Aqueous formulation according to any of claims 1 to 6, **characterised in that** the lipophilic composition has a repellent function for harmful animals.

8. Aqueous formulation according to claim 6, **characterised in that** the lipophilic composition comprises p-menthane-3,8-diol (PMD).

9. Aqueous formulation according to any of claims 1 to 8, **characterised in that** the lipophilic composition has at least one function chosen from therapeutic and/or prophylactic functions, in particular soothing functions intended to ease stings or bites of harmful animals, anti-burn, healing, relaxing and antiinflammatory functions, and wellbeing functions, in particular perfuming, deodorant, biocidal, bactericidal and fungicidal functions.

10. Aqueous formulation according to any of claims 1 to 9, **characterised in that** it comprises between 0.1% and 2% by weight of homogenising component.

11. Aqueous formulation according to any of claims 1 to 10, **characterised in that** the homogenising component comprises a mixture of microcrystalline cellulose and carboxymethylcellulose.

12. Aqueous formulation according to any of claims 1 to 11, **characterised in that** it comprises less than 1%, in particular less than 0.5%, by weight of clay.

13. Aqueous formulation according to any of claims 1 to 12, **characterised in that** it comprises between 0.1% and 50%, in particular between 5% and 20%, by weight of perfuming composition.

14. Aqueous formulation according to claim 13, **characterised in that** the perfuming composition comprises an essential oil or a mixture of essential oils.

15. Aqueous formulation according to any of claims 1 to 14, **characterised in that** it further comprises less than 20% by weight of a preservative agent.

16. Aqueous formulation according to claim 15, **characterised in that** the preservative agent comprises phenethyl alcohol of natural origin, in particular in a quantity of between 0.4% and 5% by weight of said formulation.

17. Aqueous formulation according to any of claims 1 to 16, **characterised in that** it further comprises less than 10% by weight of antioxidant agent.

18. Aqueous formulation according to claim 17, **characterised in that** the antioxidant agent comprises vitamin E, in particular in a quantity of less than 1% by weight of said formulation.

19. Aqueous formulation according to any of claims 1 to 18, **characterised in that** it further comprises less than 2% by weight of pH rectifier agent, in particular based on citric acid.

20. Aqueous formulation according to any of claims 1 to 19, **characterised in that** it further comprises between 3% and 50%, in particular less than 10%, by weight of one or more solvents, in particular chosen from alcohols, acids and/or acid salts.

21. Aqueous formulation according to claim 20, **characterised in that** the solvent(s) is(are) chosen from ethanol, isopropanol, methylenediol, ethylene glycol, propanediol, glycerol, butanediol, pentanol, isopentyldiol, hexanol, hexanediol and benzanediol.
